# EUROPÄISCHE PATENTSCHRIFT

(11) **EP 1 900 325 B1**
(45) Veröffentlichungstag und Bekanntmachung des Hinweises auf die Patenterteilung: **10.11.2010**
(21) Anmeldenummer: 07017929.6
(22) Anmeldetag: 13.09.2007
(51) Int. Cl.: A61B 5/151

(54) **Vorrichtung zur fetalen Skalpblutentnahme**
Device for taking blood from a foetal scalp
Dispositif destiné au prélèvement sanguin du cuir chevelu foetal

(30) Priorität: 13.09.2006 DE 102006042903
(43) Veröffentlichungstag der Anmeldung: 19.03.2008
(73) Patentinhaber: Karl Storz GmbH & Co. KG, 78532 Tuttlingen (DE)
(72) Erfinder: Kühfuss, Andreas, 78532 Tuttlinger (DE)
(74) Vertreter: Hofmeister, Frank

(56) Entgegenhaltungen:
- WO-A-2006/043164
- US-A- 4 360 016
- US-A- 4 653 513
- US-A- 5 908 432
- US-B1- 6 423 011

## Beschreibung

Die Erfindung betrifft eine Vorrichtung zur fetalen Skalpblutentnahme, mit einem Schaft, einer auswechselbar im distalen Ende des Schaftes gelagerten Klinge, die über einen Stellmechanismus zwischen einer im Schaft angeordneten Grundstellung und einer das distale Ende des Schaftes überragenden Arbeitsstellung verschiebbar ist, sowie mit einer am distalen Ende des Schaftes angeordneten Blutsammeleinrichtung.

Insbesondere zur Abklärung pathologischer fetaler Herzfrequenzmuster unter der Geburt hat sich die fetale Skalpblutentnahme in der Praxis bewährt. Über die entnommene Blutprobe lassen sich beispielsweise der pH-Wert, der Base-Excess-Wert sowie weitere vitale Blutparameter einfach und schnell bestimmen.

Eine gattungsgemäße Vorrichtung mit einer auswechselbar im Schaft gelagerten Klinge ist beispielsweise aus der DE 84 21 169 U1 bekannt. Bei dieser bekannten Vorrichtung ist die Klinge über einen Stempel gegen die Kraft einer Rückstellfeder in die ausgefahrene Arbeitsstellung verschiebbar. Aufgrund der Federkraft der Rückstellfeder muss der Operateur den Stempel während des Arbeitseinsatzes der Klinge fortwährend eingedrückt halten, was die Handhabbarkeit dieser Vorrichtung beeinträchtigt.

Eine weitere Vorrichtung zur fetalen Skalpblutentnahme ist beispielsweise der "Fetal Scalp Blood Sampler" der Firma Brenner Medical, Putzbrunn. Bei dieser bekannten, als Einweginstrument ausgebildeten Vorrichtung bildet die verschiebbar im Schaft gelagerte Klinge einen unlösbaren festen Bestandteil der Vorrichtung, so dass weder verschiedene Inzisionstiefen der Klinge einstellbar sind noch die Vorrichtung nach einer Reinigung wiederverwendet werden kann.

Die Dokumente US-B1-6 423 011 und US-A-4 360 016 offenbaren Vorrichtungen zur fetalen Skalpblutentnahme, mit einem Schaft, einer auswechselbar im distalen Ende des Schaftes gelagerten Klinge, die über einen Stellmechanismus zwischen einer im Schaft angeordneten Grundstellung und einer das distale Ende des Schaftes überragenden Arbeitsstellung verschiebbar ist, sowie mit einer am distalen Ende des Schaftes angeordneten Blutsammeleinrichtung.

Die genannte US-B1-6 423 011 zeigt auch einen federelastischen Druckknopf, eine distale Klingenaufnahme, ein auswechselbares Kapillarröhrchen, einen distalen Lichtleiter, und eine Stellschraube zur Einstellung der Inzisionstiefe, ' wobei die Klinge über den Stellmechanismus in der jeweiligen Endstellung fixierbar ist.

Die Dokumente WO 2006/043164 A und US-A-5 908 432 beschreiben distale Wechselstellungen von Klingen.

Dokument US-A-4 653 513 zeigt eine Vorrichtung zur Blutentnahme mit Grund-Arbeits- und Wechselstellung der Klingenaufnahme, mit einer U-förmigen Führungsschiene, und eine die Arbeitsstellung in distaler Richtung überragende Klingenwechselstellung, wobei die U-förmigen Führungsbahn unterschiedlich lang ausgebildete Schenkel aufweist.

Davon ausgehend liegt der Erfindung die **Aufgabe** zugrunde, eine Vorrichtung zur fetalen Skalpblutentnahme zu schaffen, die bei einfacher Handhabbarkeit eine hohe Betriebssicherheit gewährleistet.

Die **Lösung** dieser Aufgabenstellung ist erfindungsgemäß durch die Merkmale von Anspruch 1 gegeben.

Das Verschieben der in der Klingenaufnahme angeordneten Klinge erfolgt über einen im Schaft gelagerten Stellmechanismus. Um sicherzustellen, dass die Klinge bei der Benutzung in der jeweils gewünschten Endstellung, nämlich der Grundstellung, der Arbeitsstellung oder der Wechselstellung, verbleibt, wird mit der Erfindung vorgeschlagen, dass die Klinge über den Stellmechanismus in der jeweiligen Endstellung fixierbar ist.

Weiterhin wird mit der Erfindung vorgeschlagen, dass der Stellmechanismus einen in einer Führungsbahn verschiebbaren Druckknopf umfasst, wobei die Konturen der Führungsbahn sowie des Druckknopfschaftes so aufeinander abgestimmt sind, dass der Druckknopf in den verschiedenen Endstellungen der Klinge unverschiebbar in der Führungsbahn positioniert ist.

Ein unbeabsichtigtes Betätigen des Stellmechanismus kann erfindungsgemäß weiterhin dadurch verhindert werden, dass der Druckknopf gegen die Kraft mindestens eines federelastischen Elements in die Führungsbahn eindrückbar ist. Diese auf den Druckknopf wirkende Federkraft verhindert so einerseits ein versehentliches Verschieben des Druckknopfes durch seitliches Anstoßen und sorgt andererseits dafür, dass der Druckknopf in die verschiebesicheren Endstellungen vorgespannt ist.

Gemäß der Erfindung wird vorgeschlagen, dass die Klinge in einer in Axialrichtung verschiebbar im Schaft angeordneten Klingenaufnahme gelagert ist, wobei als Klingen vorzugsweise an sich bekannte Skalpell-Klingen Verwendung finden.

Um ein versehentliches Öffnen der Klingenaufnahme zu verhindern, ist die Klingenaufnahme erfindungsgemäß so ausgebildet, dass die Klinge ausschließlich in einer die Arbeitsstellung der Klinge in distaler Richtung überragenden Wechselstellung aus der Klingenaufnahme entnehmbar bzw. in diese einsetzbar ist. Ein unbeabsichtigtes Lösen der Klinge kann auf diese Weise ausgeschlossen werden.

Mit einer praktischen Ausführungsform zur Ausgestaltung der Klingenaufnahme wird vorgeschlagen, dass die Klingenaufnahme als maulartig ausgebildeter Klappmechanismus ausgebildet ist. Diese konstruktive Ausgestaltung stellt eine einfache und sichere Halterung dar, da die Klinge fixierend zwischen den Maulteilen eingeklemmt wird. Vorzugsweise ist die Klingenaufnahme weiterhin so ausgestaltet, dass die Inzisionstiefe der Klinge über die Anordnung der Klinge in der Klingenaufnahme einstellbar ist. Zu diesem Zweck können in der Klingenaufnahme beispielsweise verschiedene abgestufte Anlageflächen oder dergleichen ausgebildet sein, die eine exakte Einstellung der Inzisionstiefe durch entsprechende Anordnung der Klinge ermöglicht.

Zur Ausbildung der Blutsammeleinrichtung wird mit der Erfindung vorgeschlagen, dass diese als am Schaft auswechselbar festlegbares Kapillarröhrchen ausgebildet ist.

Schließlich wird mit der Erfindung vorgeschlagen, dass im Schaft ein bis zum distalen Ende des Schaftes reichender Lichtleiter angeordnet ist, um auch bei beengten Platzverhältnissen eine ausreichende Ausleuchtung des Einsatzgebietes zu gewährleisten.

Weitere Merkmale und Vorteile der Erfindung ergeben sich anhand der zugehörigen Zeichnung, in der zwei Ausführungsbeispiele einer erfindungsgemäßen Vorrichtung zu fetalen Skalpblutentnahme nur beispielhaft dargestellt sind, ohne die Erfindung auf diese Ausführungsbeispiele zu beschränken. In der Zeichnung zeigt:

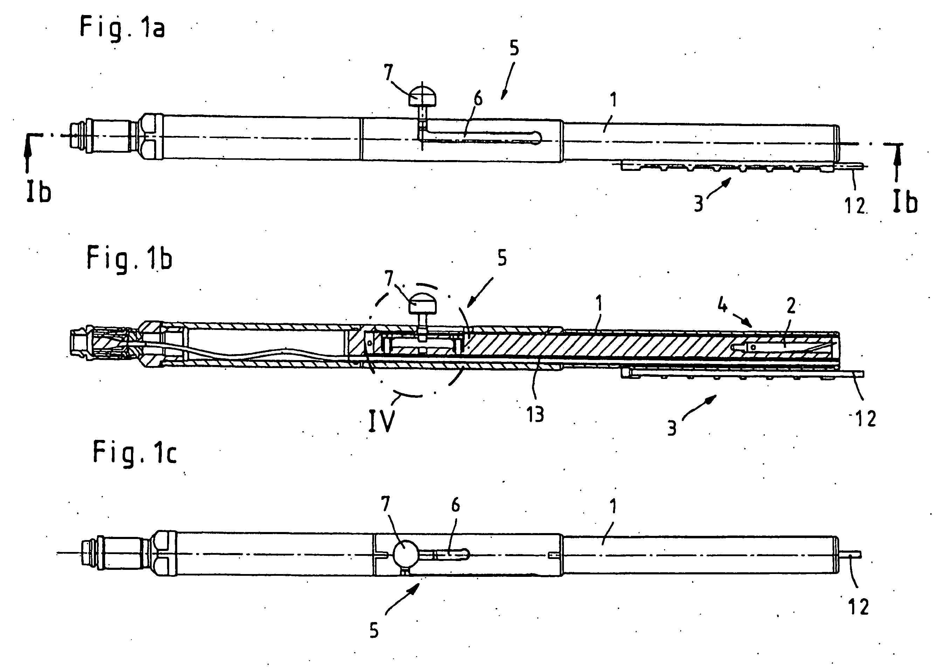

- Fig. 1a: eine Seitenansicht einer ersten Ausführungsform einer erfindungsgemäßen Vorrichtung zu fetalen Skalpblutentnahme, die Grundstellung der Klinge darstellend;
- Fig. 1 b: einen Schnitt entlang der Linie Ib-Ib gemäß Fig. 1a
- Fig. 1 c: eine Draufsicht der Abbildung gemäß Fig. 1a
- Fig. 2a: eine Seitenansicht einer erfindungsgemäßen Vorrichtung zu fetalen Skalpblutentnahme, die Arbeitsstellung der Klinge darstellend;
- Fig. 2b: eine Draufsicht der Abbildung gemäß Fig. 2a;
- Fig. 3a: eine Seitenansicht einer erfindungsgemäßen Vorrichtung zu fetalen Skalpblutentnahme, die Wechselstellung der Klinge darstellend;
- Fig. 3b: eine Draufsicht der Abbildung gemäß Fig. 3a;
- Fig. 4: eine vergrößerte Detailansicht des Details IV gemäß Fig. 1b und
- Fig. 5: eine Fig. 1b entsprechende Schnittdarstellung, jedoch eine zweite Ausführungsform einer erfindungsgemäßen Vorrichtung zu fetalen Skalpblutentnahme darstellend.

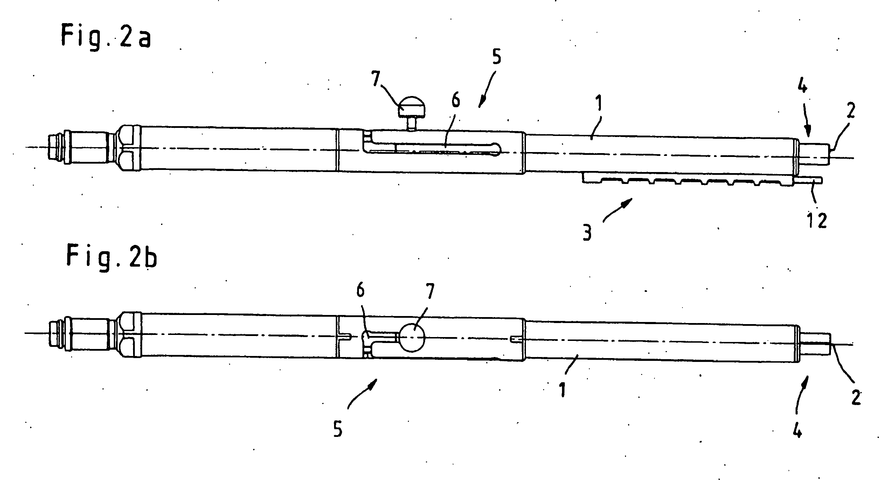

Die in den Abbildungen Fig. 1 bis Fig. 3b schematisch dargestellte Vorrichtung zu fetalen Skalpblutentnahme besteht im Wesentlichen aus einem rohrförmigen Schaft 1, einer in Axialrichtung des Schaftes 1 verschiebbar im distalen Ende des Schaftes 1 gelagerten Klinge 2 sowie einer am distalen Ende des Schaftes 1 angeordneten Blutsammeleinrichtung 3.

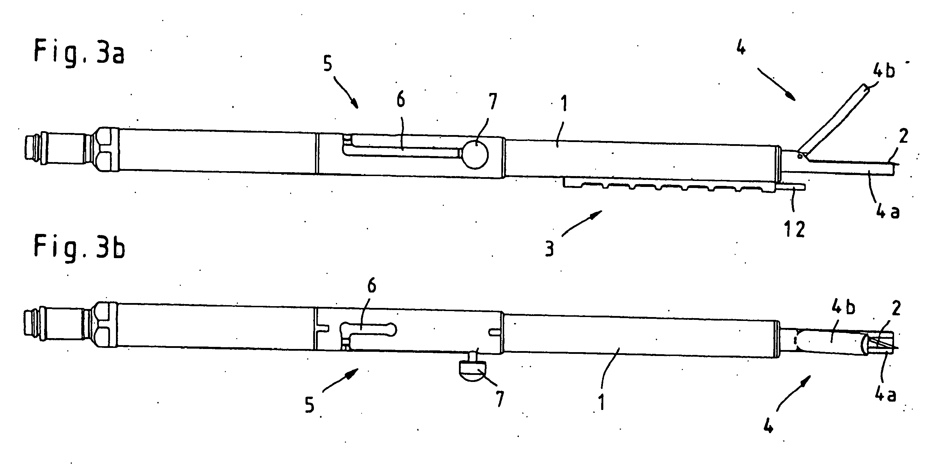

Wie aus den Abbildungen Fig. 1b, 3a und 3b ersichtlich, ist die Klinge 2 auswechselbar in einer Klingenaufnahme 4 gelagert, die über einen Stellmechanismus 5 in Axialrichtung des Schaftes 1 verschiebbar im Schaft 1 gelagert ist. Bei der dargestellten Ausführungsform ist die Klingenaufnahme 4 maulartig so ausgebildet, dass das distale Ende der Klingenaufnahme 4 einen starren Grundkörper 4a zur lagernden Aufnahme der Klinge 2 sowie einen gegenüber dem starren Grundkörper 4a verschwenkbaren Deckel 4b aufweist.

In der in Fig. 3a dargestellten Wechselstellung wurde die Klingenaufnahme 4 über den Stellmechanismus 5 distalseitig soweit aus dem Schaft 1 herausgeschoben, bis der Deckel 4b der Klingenaufnahme 4 vom starren Grundkörper 4a fort aufgeklappt werden kann, um eine alte Klinge 2 zu entnehmen und eine neue Klinge 2 in der Klingenaufnahme 4 anzuordnen. Wie aus den Abbildungen 1 b, 2a, 2b sowie 3a und 3b ersichtlich, ist die Klinge 2 immer so in der Klingenaufnahme 4 angeordnet, dass die distale Spitze der Klinge 2 aus dem distalseitigen Ende der Klingenaufnahme 4 herausragt.

Das Maß, um das die Klinge 2 das distalseitige Ende der Klingenaufnahme 4 überragt, entspricht der Inzisionstiefe der Klinge 2 bei der Blutentnahme. Durch entsprechende Anschläge in der Klingenaufnahme 4 ist der Überstand der Klinge 2 und somit die Inzisionstiefe der Klinge durch entsprechende Positionierung der Klinge 2 in der Klingenaufnahme 4 einstellbar.

Der Stellmechanismus 5, über den die mit der Klinge 2 bestückte Klingenaufnahme 4 in Axialrichtung des Schaftes 1 innerhalb des Schaftes 1 verschiebbar ist, besteht bei der dargestellten Ausführungsform aus einem in einer im Schaft 1 ausgebildeten Führungsbahn 6 verschiebbaren Druckknopf 7. Wie aus der Zusammenschau der Seitenansichten und Draufsichten gemäß Fig. 1a und 1c, 2a und 2b sowie 3a und 3b ersichtlich, ist die Führungsbahn 6 im Wesentlichen U-förmig mit zwei parallel in Axialrichtung des Schaftes 1 verlaufenden Schenkeln ausgebildet, wobei der offene Durchmesser der Führungsbahn 6 an drei Stellen vergrößert ausgebildet ist, nämlich zum einen an den beiden freien, im Bezug auf das Gesamtinstrument distalen Enden der parallelen Schenkel der U-förmigen Führungsbahn 6 sowie an einem Knick im Übergang von der quer verlaufenden Basis der U-förmigen Führungsbahn 6 zu einem Schenkel.

Diese drei Positionen mit vergrößertem Führungsbahndurchmesser markieren Endstellungen der Klingenaufnahme 4 und somit auch der Klinge 2 bezüglich deren Lage zum Schaft 1.

In der in Fig. 1a bis 1c dargestellten Grundstellung ist die Klingenaufnahme 4 über den Stellmechanismus 5 soweit in den Schaft 1 eingezogen, dass die Klinge 2 vollständig Aufnahme im Schaft 1 findet und keine Verletzungsgefahr von der die Klingenaufnahme 4 distalseitig überragenden Klinge 2 ausgehen kann. Die entsprechende Endstellung des Druckknopfes 7 ist die Position im Übergang von der quer verlaufenden Basis der U-förmigen Führungsbahn 6 zu einem Schenkel, also die am weitesten zum proximalseitigen Ende des Schaftes 1 hin verlagerte Position des Druckknopfes 7.

Um die Klingenaufnahme 4 durch Axialverschieben einerseits in die Arbeitsstellung und andererseits in die Wechselstellung überführen zu können, sind die beiden parallelen Schenkel der U-förmigen Führungsbahn 6 unterschiedlich lang ausgebildet.

Das Überführen der Klingenaufnahme 4 in die Arbeitsstellung erfolgt durch Verschieben des Druckknopfes 7 bis zum Ende des kurzen Schenkels. In dieser Position überragen die Klingenaufnahme 4 und die aus der Klingenaufnahme 4 herausragende Spitze der Klinge 2 das distalseitige Ende des Schaftes 1 soweit, dass das Andrücken der Vorrichtung gegen die Kopfhaut des Patienten zum Einstechen der Klingenspitze in die Kopfhaut führt.

Die in Fig. 3a und 3b dargestellte Wechselstellung, bei der sich der Druckknopf 7 des Stellmechanismus 5 am Ende des langen Schenkels der U-förmigen Führungsbahn 6 befindet, unterscheidet sich von der Arbeitsstellung dadurch, dass die Klingenaufnahme 4 mittels des Stellmechanismus 5 deutlich über die Arbeitsstellung hinaus aus dem distalseitigen Ende des Schaftes 1 herausgeschoben wurde, bis der Deckel 4b der Klingenaufnahme 4 zum Austausch der Klinge 2 geöffnet werden kann.

Um sicherzustellen, dass die Klingenaufnahme 4 und somit auch die Klinge 2 in der jeweiligen Endstellung fixiert ist und der Druckknopf 7 nicht unbeabsichtigt verschoben werden kann, sind die Konturen der Führungsbahn 6 sowie des Druckknopfschaftes so aufeinander abgestimmt sind, dass der Druckknopf 7 in den verschiedenen Endstellungen der Klingenaufnahme 4 unverschiebbar in der Führungsbahn 6 positioniert ist. Zu diesem Zweck weist der Schaft des Druckknopfes 7 eine Verdickung auf, die exakt mit der Vergrößerung des Führungsbahndurchmessers im Bereich der Endstellungen korrespondiert. In dieser Stellung, in der der verdickte Bereich des Druckknopfschaftes Aufnahme in dem vergrößerten Führungsbahndurchmesser findet, ist ein bloßes seitliches Verschieben des Druckknopfes 7 nicht mehr möglich.

Ein erneutes Verschieben des Druckknopfes 7 ist erst wieder möglich, wenn der Druckknopf nach unten in die Führungsbahn 6 hinein gedrückt wird, bis der verdickte Bereich außer Eingriff mit der Führungsbahn 6 tritt.

Ein unbeabsichtigtes Betätigen des Stellmechanismus 5 wird weiterhin dadurch verhindert, dass der Druckknopf 7 gegen die Kraft mindestens eines federelastischen Elements 8 in die Führungsbahn 6 eindrückbar ist. Diese auf den Druckknopf 6 wirkende Federkraft verhindert so einerseits ein versehentliches Verschieben des Druckknopfes 6 durch seitliches Anstoßen und sorgt andererseits dafür, dass der Druckknopf 6 in die einzelnen verschiebesicheren Endstellungen vorgespannt ist.

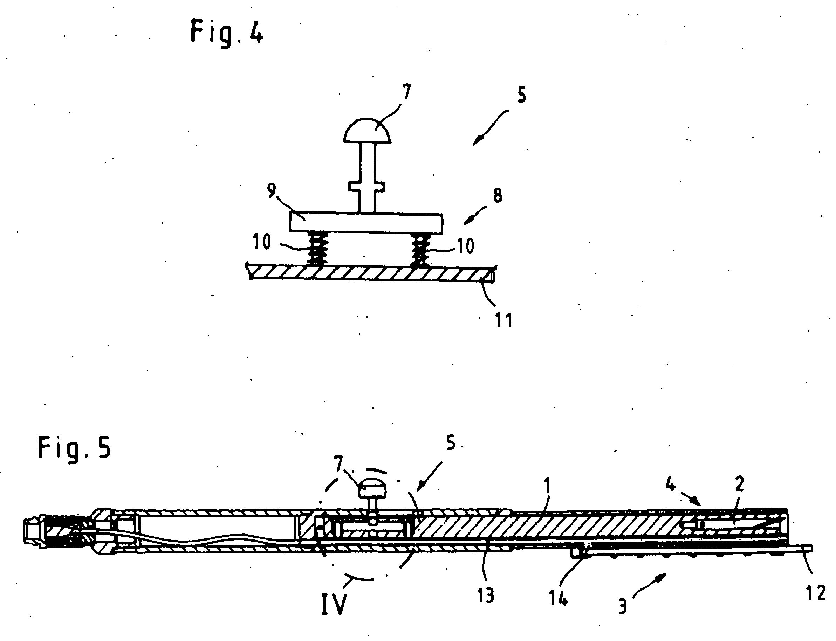

Wie aus Fig. 4 ersichtlich, ist das den Druckknopf 6 vorspannende federelastische Element 8 bei der dargestellten Ausführungsform so ausgebildet, dass der Schaft des Druckknopfes 6 auf einer Grundplatte 9 angeordnet ist, die sich über zwei Federelemente 10 auf einer Zug-/Schubstange 11 des Stellmechanismus 5 abstützt.

Wie weiterhin insbesondere aus den Seitenansichten gemäß Fig. 1a, 2a und 3a ersichtlich, besteht die Blutsammeleinrichtung 3 bei der dargestellten Ausführungsform aus einem seitlich am distalen Ende des Schaftes 1 auswechselbar festlegbaren Kapillarröhrchen 12, das die Blutprobe mittels Kapillareffekt aufsaugt.

Um auch bei beengten Platzverhältnissen eine jederzeit ausreichende Ausleuchtung des Einsatzgebietes zu gewährleisten, ist im Schaft 1 darüber hinaus ein bis zum distalen Ende reichender Lichtleiter 13 angeordnet, der in unmittelbarer Nähe der Klinge 2 sowie des Kapillarröhrchens 12 das Einsatzgebiet ausleuchtet.

Die in Fig. 5 dargestellte zweite Ausführungsform der Vorrichtung zu fetalen Skalpblutentnahme unterscheidet sich von der zuvor beschriebenen Ausführungsform dadurch, dass derselbe Lichtleiter 13 oder ein weiterer Lichtleiter 13 eine Lichtaustrittsöffnung 14 aufweist, über die zusätzlich das proximale Ende des Kappilarröhrchens 12 beleuchtbar ist. Diese zusätzliche Beleuchtung des proximalen Endes des Kappilarröhrchens 12 ist für den Operateur vorteilhaft, da er so feststellen kann, ob aufgrund der wirkenden Kappilarkräfte eine für die nachfolgenden Untersuchungen ausreichende Blutmenge in das Kappilarröhrchen 12 aufgenommen wurde.

Eine solchermaßen ausgestaltete Vorrichtung zu fetalen Skalpblutentnahme zeichnet sich dadurch aus, dass die Vorrichtung durch die Auswechselbarkeit der Klinge 2 als wiederverwendbares Mehrweginstrument ausgebildet ist.

Zu diesem Zweck ist die Vorrichtung selbstverständlich durch entsprechende Materialauswahl und Abdichtungsmaßnahmen so auszugestalten, dass die Vorrichtung vollständig zerlegbar und zu Reinigungszwecken autoklavierbar ist.

Ein weiterer Vorteil der voranstehend beschriebenen Vorrichtung besteht darin, dass die Klinge 2 über den Stellmechanismus 5 in definierte und fixierbare Endstellungen überführbar ist, die eine hohe Betriebssicherheit gewährleisten.

### Bezugszeichenliste

- 1: Schaft
- 2: Klinge
- 3: Blutsammeleinrichtung
- 4: Klingenaufnahme
- 4a: starrer Grundkörper
- 4b: Deckel
- 5: Stellmechanismus
- 6: Führungsbahn
- 7: Druckknopf
- 8: federelastisches Element
- 9: Grundplatte
- 10: Federelement
- 11: Zug-/Schubstange
- 12: Kapillarröhrchen
- 13: Lichtleiter
- 14: Lichtaustrittsöffnung

## Patentansprüche

1. Vorrichtung zur fetalen Skalpblutentnahme, mit einem Schaft (1), einer auswechselbar im distalen Ende des Schaftes (1) gelagerten Klinge (2), die in einer in Axialrichtung verschiebbar im Schaft (1) angeordneten Klingenaufnahme (4) gelagert ist und über einen mit einer Führungsbahn (6) versehenen Stellmechanismus (5) zwischen einer im Schaft (1) angeordneten Grundstellung, einer das distale Ende des Schaftes (1) überragenden Arbeitsstellung sowie einer die Arbeitsstellung in distaler Richtung überragenden Wechselstellung verschiebbar ist, wobei die Klinge (2) über den Stellmechanismus (5) in einer Endstellung fixierbar ist, sowie mit einer am distalen Ende des Schaftes (1) angeordneten Blutsammeleinrichtung (3),
**dadurch gekennzeichnet,**
**dass** der Stellmechanismus (5) einen in einer U-förmig ausgebildeten Führungsbahn (6) verschiebbaren federbelasteten Druckknopf (7) umfasst, wobei die beiden in Axialrichtung des Schaftes (1) verlaufenden Schenkel der U-förmigen Führungsbahn (6) unterschiedlich lang ausgebildet sind und, dass die Klinge (2) über den Stellmechanismus (5) in jeder Endstellung fixierbar ist.

2. Vorrichtung nach Anspruch 1, **dadurch gekennzeichnet, dass** die Konturen der Führungsbahn (6) sowie des Druckknopfschaftes so aufeinander abgestimmt sind, dass der Druckknopf (7) in den verschiedenen Endstellungen der Klinge (2) unverschiebbar in der Führungsbahn (6) positioniert ist.

3. Vorrichtung nach Anspruch 2, **dadurch gekennzeichnet, dass** der Druckknopf (7) gegen die Kraft mindestens eines federelastischen Elements (8) in die Führungsbahn (6) eindrückbar ist.

4. Vorrichtung nach einem der ansprüche 1 bis 3, **dadurch gekennzeichnet, dass** die Klinge (2) ausschließlich in der die Arbeitsstellung in distaler Richtung überragenden Wechseistellung aus der Klingenaufnahme (4) entnehmbar bzw. in diese einsetzbar ist.

5. Vorrichtung nach einem der Ansprüche 1 bis 4, **dadurch gekennzeichnet, dass** die Klingenaufnahme (4) als maulartig ausgebildeter Klappmechanismus ausgebildet ist.

6. Vorrichtung nach einem der Ansprüche 1 bis 5, **dadurch gekennzeichnet, dass** die Inzisionstiefe der Klinge (2) über die Anordnung der Klinge (2) in der Klingenaufnahme (4) einstellbar ist.

7. Vorrichtung nach einem der Ansprüche 1 bis 6, **dadurch gekennzeichnet, dass** die Blutsammeleinrichtung (3) als am Schaft (1) auswechselbar festlegbares Kapillarröhrchen (12) ausgebildet ist.

8. Vorrichtung nach einem der Ansprüche 1 bis 7, **dadurch gekennzeichnet, dass** im Schaft (1) ein bis zum distalen Ende des Schaftes (1) reichender Lichtleiter (13) angeordnet ist.

## Claims

1. Device for taking blood from a foetal scalp, with a shaft (1), with a blade (2) which is mounted exchangeably in the distal end of the shaft (1), and is mounted in a blade holder (4) arranged axially movably in the shaft (1), and which, by means of an adjusting mechanism (5) provided with a guide track (6), can be moved between a starting position arranged in the shaft (1), a working position extending beyond the distal end of the shaft (1), and an exchange position extending in the distal direction beyond the working position, said blade (2) being able to be fixed in an end position via the adjusting mechanism (5), and with a blood sampler (3) arranged at the distal end of the shaft (1), **characterized in that** the adjusting mechanism (5) comprises a spring-loaded push-button (7) that can be moved in a U-shaped guide track (6), wherein the two branches of the U-shaped guide track (6) that extend in the axial direction of the shaft (1) are configured with different lengths, and the blade (2) can be fixed in each end position via the adjusting mechanism (5).

2. Device according to Claim 1, **characterized in that** the contours of the guide track (6) and of the push-button shaft are adapted to each other in such a way that the push-button (7) is positioned immovably in the guide track (6) in the various end positions of the blade (2).

3. Device according to Claim 2, **characterized in that** the push-button (7) can be pressed into the guide track (6) counter to the force of at least one spring-elastic element (8).

4. Device according to one of Claims 1 to 3, **characterized in that** the blade (2) can be removed from or inserted into the blade holder (4) only in the exchange position extending in the distal direction beyond the working position.

5. Device according to one of Claims 1 to 4, **characterized in that** the blade holder (4) is configured as a jaw-like flap mechanism.

6. Device according to one of Claims 1 to 5, **characterized in that** the depth of incision of the blade (2) can be adjusted by the arrangement of the blade (2) in the blade holder (4).

7. Device according to one of Claims 1 to 6, **characterized in that** the blood sampler (3) is configured as a capillary tube (12) that can be secured exchangeably on the shaft (1).

8. Device according to one of Claims 1 to 7, **characterized in that** a light guide (13) is arranged in the shaft (1) and reaches as far as the distal end of the shaft (1).

## Revendications

1. Dispositif pour effectuer un prélèvement sanguin foetal au scalp ou sur cuir chevelu, comprenant un corps allongé (1), une lame de coupe (2) agencée de manière interchangeable à l'extrémité distale du corps allongé (1), et montée dans un logement de support de lame de coupe (4) agencé de manière à pouvoir coulisser en direction axiale dans le corps allongé (1), la lame de coupe étant susceptible de coulisser, au moyen d'un mécanisme de déplacement (5) muni d'une glissière de guidage (6), entre une position de base où elle est logée dans le corps allongé (1), une position de travail dans laquelle elle fait saillie de l'extrémité distale du corps allongé (1), et une position de remplacement de lame située au-delà de la position de travail en direction distale, et la lame de coupe (2) pouvant être immobilisée dans une position extrême par l'intermédiaire du mécanisme de déplacement (5), le dispositif comprenant en outre un ensemble de collecte de sang (3) agencé à l'extrémité distale du corps allongé (1),
**caractérisé en ce que** le mécanisme de déplacement (5) comprend un bouton poussoir (7) chargé par ressort et pouvant coulisser dans une glissière de guidage (6) présentant une configuration en forme de U, les deux branches ou ailes du U de la glissière de guidage (6) en forme de U, qui s'étendent dans la direction axiale du corps allongé (1), étant réalisées de longueur différente, et **en ce que** la lame de coupe (2) peut être immobilisée dans chaque position extrême par l'intermédiaire du mécanisme de déplacement (5).

2. Dispositif selon la revendication 1, **caractérisé en ce que** les contours de la glissière de guidage (6) ainsi que de la tige de bouton poussoir sont adaptés réciproquement l'un à l'autre, de façon à ce que le bouton poussoir (7) soit positionné de manière non coulissante dans la glissière de guidage (6), dans les différentes positions extrêmes de la lame de coupe (2).

3. Dispositif selon la revendication 2, **caractérisé en ce que** le bouton poussoir (7) peut être repoussé dans la glissière de guidage (6) à l'encontre de la force d'au moins un élément de ressort élastique (8).

4. Dispositif selon l'une des revendications 1 à 3, **caractérisé en ce que** la lame de coupe (2) peut être insérée dans le logement de support de lame de coupe (4) ou être extraite de celui-ci, uniquement dans la position de remplacement de lame située au-delà de la position de travail en direction distale.

5. Dispositif selon l'une des revendications 1 à 4, **caractérisé en ce que** le logement de support de lame de coupe (4) est réalisé en tant que mécanisme à mâchoire rabattable.

6. Dispositif selon l'une des revendications 1 à 5, **caractérisé en ce que** la profondeur d'incision de la lame de coupe (2) peut être réglée par l'agencement de la lame de coupe (2) dans le logement de support de lame de coupe (4).

7. Dispositif selon l'une des revendications 1 à 6, **caractérisé en ce que** l'ensemble de collecte de sang (3) est réalisé sous la forme d'un petit tube capillaire (12) pouvant être fixé de manière interchangeable sur le corps allongé (1).

8. Dispositif selon l'une des revendications 1 à 7, **caractérisé en ce que** dans le corps allongé (1) est agencé un guide de lumière (13) ou fibre optique, qui s'étend jusqu'à l'extrémité distale du corps allongé (1).
